# EUROPEAN PATENT SPECIFICATION

(11) **EP 2 803 276 B1**
(45) Date of publication and mention of the grant of the patent: **29.07.2015**
(21) Application number: 14162467.6
(22) Date of filing: 28.03.2014
(51) Int. Cl.: A23K 1/16, A23K 1/18, A61P 1/00, A23K 1/00

(54) **Product for the diet of dogs afected by inflammatory intestinal diseases**
Produkt für die Ernährung von Hunden mit entzündlichen Darmerkrankungen
Produit pour l'alimentation des chiens atteints de maladies intestinales inflammatoires

(30) Priority: 14.05.2013 IT PD20130133
(43) Date of publication of application: 19.11.2014
(73) Proprietor: Sanypet S.p.a., 35023 Bagnoli di Sopra (PD) (IT)
(72) Inventor: Canello, Sergio, I-35037 Villa di Teolo (PD) (IT); Pengo, Graziano, I-26012 Castelleone (CR) (IT); Guidetti, Gianandrea, I-42121 Reggio Emilia (RE) (IT)
(74) Representative: Gallo, Luca

(56) References cited:
- JP-A- 2007 284 361
- US-A1- 2003 049 240
- US-A1- 2003 195 166
- US-A1- 2006 198 817
- US-A1- 2009 148 414
- US-A1- 2012 294 954
- US-B1- 6 312 709
- US-B1- 6 391 331
- US-B1- 6 403 142
- DATABASE GNPD [Online] MINTEL; 30 June 2012 (2012-06-30), Anonymus: "Super Premium Junior Dog Food", XP002717557, Database accession no. 1824772

## Description

### Field of application

The present invention regards a product for the diet of dogs affected by inflammatory intestinal diseases according to the preamble of the independent claim No. 1.

The product, object of the present invention, is intended to be advantageously administered to dogs with the purpose of restoring the intestinal equilibrium compromised by acute or chronic inflammatory conditions.

The invention is therefore inserted in the field of the industry for the production of products for the diet and treatment of domestic animals, and in particular of dogs.

### State of the art

In the field of production of products for the diet and treatment of animals, there has been for some time the need to provide specific products dedicated to the diet of animals affected by diseases of various nature, or more generally of animals whose organism has non-normal physiological conditions and hence which require a controlled diet.

Such animal diet products must bring the nutritional substances that the animal needs, preferably in a form easily assimilable by the animal, without compromising and possibility facilitating the restoration of optimal physiological conditions of the animal organism itself.

For example, specific products which are dedicated to the diet of dogs affected by inflammatory intestinal diseases have been available on the market for years.

Such products generally prefer sources of proteins considered to have lower allergenic power and be more easily digestible, for example white meats, such as chicken, turkey and rabbit, and have a limited supply of fats.

Indeed, as is known, the animals affected by the aforesaid disturbances are more inclined to develop food intolerances, since the intestinal inflammations cause an alteration in the response of the immune system, which becomes particularly sensitive.

The patent WO 2004/056198 describes, for example, a moist or semi-moist product for the diet of dogs adapted to improve or preserve the intestinal health thereof. Such product is in particular indicated for the diet of dogs affected by food intolerances and comprises a source of rice starch, a source of fermentable fibers and a source of non-fermentable fibers, the latter preferably respectively constituted by sugar beet pulp and by cellulose. The product described herein can also comprise one or more sources of proteins, such as pieces of meat or animal derivatives or protein-rich vegetables, soy proteins, milk proteins or gluten proteins. Nevertheless, such product is limited to preserving the intestinal health condition of the dog who swallows it, or to mildly improve the condition thereof, but it is not capable of actively restoring over time the optimal physiological conditions of the dog's digestive apparatus, and in particular of the dog's intestine.

In addition, the above-described product, if administered to dogs affected by inflammatory intestinal diseases, could cause an aggravation of the pathological condition thereof.

The ingredients comprised in the above-described product can in fact stimulate an allergenic response by the immune system and thus cause a recurrence of the intestinal inflammation as well as cause the development of new food intolerances or aggravate those already existing.

Finally, the canine diet product described briefly above may not be very appealing/tasty for dogs, especially if it contains high quantities of fibers brought by cellulose.

Also known from the patent US 6 342 242 is the treatment of pasture lands, intended to be subsequently grazed on by cattle, with an extract of algae, in particular of the species Ascophyllum nodosum, or to introduce such algae directly into the cattle feed in a weight percentage up to a maximum of 3% with respect to the total weight of the feed dose administered to the animals. According to that reported in the abovementioned patent, algae have the capacity to reinforce the immune system of the animals that take it.

Nevertheless, the tests reported in the aforesaid patent have shown that the assumption by the cattle of feed with added algae is lower than the assumption of non-modified feed, an indication therefore of the lower appeal of the feed containing algae for the cattle.

### Presentation of the invention

In this situation, the problem underlying the present invention is to provide a product for the diet of dogs affected by inflammatory intestinal diseases, which is capable of actively facilitating the restoration of the optimal physiological conditions of the digestive apparatus and in particular of the intestine of the dogs that assume such product.

Another object of the present invention is to provide a product for the diet of dogs affected by inflammatory intestinal diseases, which is capable of performing an anti-inflammatory action on the intestinal apparatus.

A further object of the present invention is to provide a product for the diet of dogs affected by inflammatory intestinal diseases, which is well tolerated by the dogs themselves and is minimally allergenic.

Another object of the present invention is to provide a product for the diet of dogs affected by inflammatory intestinal diseases, which ensures an optimal assimilation of the nutrient substances contained therein.

A further object of the present invention is to provide a product for the diet of dogs affected by inflammatory intestinal diseases, which is appealing/tasty for dogs. These and other objects are all attained by the animal diet product according to the enclosed claims.

The technical characteristic of the invention, according to the aforesaid objects, can be clearly found in the contents of the below-reported claims, and the advantages thereof are more evident in the following description made with reference to a merely exemplifying and non-limiting embodiment.

### Brief description of the drawings

The technical characteristics of the invention, according to the aforesaid objects, can be clearly seen in the contents of the below-reported claims and the advantages thereof are more evident in the following detailed description, made with reference to the enclosed drawings, which refer to a merely exemplifying and non-limiting embodiment, in which:
- Figure 1 shows a first graph reporting the number of cases resolved and the number of cases not resolved (the latter reported in the upper portion with different color of each of the histogram rectangles) with the administration of the canine diet product according to the present invention for each of the pathologies considered and for all of the subjects on which the clinical tests described below were conducted;
- Figure 2 shows a second graph reporting the percentage of cases resolved with the administration of the canine diet product according to the present invention for the different considered pathologies and for all the subjects on which the clinical tests were conducted;
- Figure 3 shows a third graph reporting the percentages of resolution of the various pathologies for the subjects on which the clinical tests were conducted, as a function of the subject's age.

### Detailed description

The product, object of the present invention, is intended to be employed for the diet of dogs affected by inflammatory intestinal diseases and is adapted to facilitate the restoration of the optimal physiological conditions of the digestive apparatus, and in particular of the intestine, of the dogs which assume it.

The product for the diet of dogs affected by inflammatory intestinal diseases in accordance with the invention comprises at least one source of dietary fiber and at least one source of protein.

In accordance with the idea underlying the present invention, the source of dietary fiber comprises ascophyllum nodosum, which is contained in the product according to the invention in a weight percentage comprised between 15% and 25%, and the source of proteins comprises one or more protein hydrolysates, which are contained in the canine diet product in a weight percentage comprised between 18% and 25% with respect to the overall weight of the product.

Preferably, ascophyllum nodosum is contained in the product in accordance with the present invention in a weight percentage equal to about 20%, where with the term "about" it is intended to indicate that the content of ascophyllum can vary between 19% and 21%.

Such high content of fiber, of the type brought by ascophyllum nodosum, facilitates the rebalancing of the intestinal flora.

Ascophyllum nodosum, in fact, brings both soluble fiber, otherwise termed fermentable fiber, and insoluble fiber, otherwise termed non-fermentable fiber. In particular, approximately 22-33% of the dietary fiber brought by ascophyllum nodosum is insoluble fiber.

The insoluble fiber portion is adapted to act mechanically on the intestinal walls, brushing them. In such a manner, the insoluble fiber removes the excess intestinal flora and prevents an irregular growth of the intestinal flora itself, which is generally a direct consequence of the alteration of the immune system caused by the inflammatory condition of the intestine.

The excess intestinal flora would in fact give rise to irregular fermentations, which would produce toxic substances and gases potentially damaging for the intestine and in general for the organism.

Due to the action of mechanical removal of the excess intestinal flora by the insoluble portion of the fiber brought by the ascophyllum, the inflammatory response of the intestine against excess intestinal flora itself is reduced, and this facilitates a regression of the pathological condition.

The mechanical action performed by the insoluble fiber portion of ascophyllum nodosum present in the product according to the present invention also facilitates a quick replacement of the intestinal epithelia, which generally occurs within a period of time of about 20-21 days.

A lower fiber content, as present in other products on the market, would not be capable of ensuring a mechanical action as effective on the intestinal walls and a replacement with the same speed of the intestinal epithelia.

The soluble fiber portion brought at the same time by ascophyllum nodosum ferments in the intestinal section and provides nourishment for the intestinal flora, and consequently for the colonocytes, in such a manner facilitating the restoration of the intestinal equilibrium.

In particular, the insoluble fiber and the soluble fiber brought by the ascophyllum nodosum, contrary to the fibers brought by other sources, are capable of carrying out the aforesaid mechanical action of brushing the intestinal walls in an effective manner, nevertheless without being particularly aggressive, i.e. without causing inflammatory reactions of the intestine for the grounds reported hereinbelow. Ascophyllum nodosum is rich with alginates, mucilage and silica, which is in particular concentrated inside the typical nodes of this alga.

The alginates and mucilage, when hydrated inside the intestine, tend to be swelled, substantially forming a hydrogel that incorporates at its interior the insoluble fiber and the silica. Such hydrogel, during its transit along the intestine, hits the intestinal walls, pushing the insoluble fiber and the silica transported thereby against such walls. In such a manner, the insoluble fiber and the silica carry out the abovementioned mechanical brushing action of the intestinal walls, which is however attenuated in the presence of the hydrogel, thus resulting less aggressive. In addition, the ascophyllum nodosum contains components provided with antioxidant properties, such as fucoxanthin and fucoidans, which carry out an anti-inflammatory action on the intestinal mucous membrane.

The actions of the two components of the ascophyllum nodosum are susceptible to produce the above-considered useful effects only in the above-indicated high quantity and in synergy with the protein hydrolysates, as reported hereinbelow. The protein hydrolysates present in the animal diet product according to the present invention are indispensable for ensuring the restoration of the intestine's normal physiological conditions.

Given their low molecular weight, protein hydrolysates are incapable of stimulating allergenic responses by the altered immune system of the dogs affected by inflammatory intestinal diseases.

The dogs which assume the product according to the present invention are therefore capable of assimilating proteins from the protein hydrolysates, without however the latter stimulating an inflammatory reaction of the immune system.

In such a manner, the restoration of the intestine's normal physiological conditions is facilitated.

It has been established, as better specified hereinbelow, that the use of a source of proteins different from protein hydrolysates - even if selected from the sources of proteins considered to have lower sensitizing power and more easily digestible, such as white chicken, turkey or rabbit meats - can negatively affect the effectiveness of the canine diet product according to the present invention.

In accordance with a preferred embodiment, the protein hydrolysates comprise fish hydrolysates. Indeed, it has been verified that the product according to the present invention containing fish hydrolysates shows greater effectiveness in facilitating the restoration of the optimal physiological conditions of the intestine of the dog that assumes the product.

Preferably, the protein hydrolysates present in the product according to the invention have a maximum molecular mass of 6,000 Dalton.

A high fiber content in a canine diet product generally means poor appeal of the product itself for the dogs, and consequently a difficult administration of the product itself. In addition, protein hydrolysates typically have a bitter taste, this also not contributing to the product appeal for dogs.

Surprisingly, it was found that the combination of ascophyllum nodosum and protein hydrolysates renders the product according to the present invention particularly attractive/tasty for dogs.

The product for the diet of dogs affected by inflammatory intestinal diseases according to the invention also advantageously contains one or more sources of carbohydrates and one or more sources of fats.

In particular the present product has an overall nutritional profile formed at least by proteins, fats, carbohydrates, dietary fiber and ash, in which the proteins are in a weight quantity comprised between 15% and 40%, the fats are in a weight quantity comprised between 5% and 25%, the carbohydrates are in a weight quantity comprised between 18% and 65%, the dietary fiber is in a weight quantity comprised between 18% and 25% and the ash is in a weight quantity lower than 10%.

In accordance with a preferred embodiment of the product according to the present invention, at least 80% of said dietary fiber is brought by ascophyllum nodosum, in order to ensure that the ingested product is capable of carrying out the above-described mechanical action on the intestinal walls.

In addition, at least 5% of the overall dietary fiber is preferably insoluble fiber.

Advantageously, the product according to the present invention also comprises one or more prebiotic substances, such as MOS (mannan-oligosaccharides) or FOS (fructo-oligosaccharides), which, due to intestinal fermentation processes, nourish the colonocytes, thus facilitating the restoration of the intestinal equilibrium. Preferably, the product for the diet of dogs affected by inflammatory intestinal diseases according to the invention is a dry food, i.e. it has a moisture content lower than 15%.

In accordance with a preferred embodiment thereof, the product according to the invention is in croquette form. With the term 'croquette' it must be generically intended any agglomerate of ingredients in compact form, comprising herein also tablets, or biscuits or granules, without departing from the protective scope of the present patent.

Preferably, the croquettes are obtained via extrusion, in particular starting from a paste of components inserted in an extrusion chamber, forced by the action of at least one worm screw to pass through the holes of a die on the head of the extruder and then cut by knives into the desired croquette form.

### Examples

Hereinbelow, we report as a non-limiting example the results of clinical tests performed on 206 dogs, of different races and ages, affected by inflammatory intestinal diseases.

The evaluation of the health condition of each of the subjects involved was of clinical-symptoms type; only in the most serious cases was a clinical evaluation with endoscopic diagnostic support carried out.

The symptoms presented by each of the subjects involved before the start of the administration of the canine diet product according to the present invention. The symptoms of the subjects were monitored during the entire period of administration of the aforesaid product.

The pathologies were considered resolved with the disappearance of the initial symptoms.

More in detail, the subjects were subjected to an exclusive diet treatment with the canine diet product, object of the present invention. 15 days after the start of the aforesaid diet treatment, 90.3% of dogs were cleared of the pathology with which they were affected.

Reported hereinbelow are the results obtained for each of the pathologies which were treated with the animal diet product according to the present invention.

### Example 1:

A first group of 19 dogs affected by colitis were fed for a month with a diet product in accordance with the present invention.

The subjects initially had one or more of the classic symptoms of the colitis pathology (diarrhea, possible presence of mucus and of blood in the feces, vomit, dysorexia or anorexia, abdominal pain, lethargy, flatulence and/or weight loss).

In a time period comprised between 1 and 20 days, the symptoms manifested before the start of the treatment disappeared in all the subjects subjected to dietary treatment with the product in accordance with the present invention.

More in detail, in all the subjects, the diarrhea symptoms disappeared, and a normal intestinal transit began anew with defecations with compact feces, not more than 2-3 per day, and urgent nature of the defecations has disappeared, with improved retention capacities.

The presence of mucus or blood in the feces is no longer encountered. In the subjects in question, there was also a disappearance of the abdominal pain, a renewal of the normal daily activity, and an appetite renewal and a consequent body weight gain.

The tests conducted on subjects affected by colitis have therefore shown that the exclusive administration of the product according to the invention has proven to resolve the disease in 100% of the cases.

### Example 2:

A second group of 139 of dogs affected with enterocolitis were fed for one month with a diet product in accordance with the present invention.

The subjects initially had one or more of the symptoms linked to the aforesaid pathology (in addition to the above-reported symptoms normally encountered in colitis pathology, the subjects affected by enterocolitis can suffer from dehydration, sadness and/or hyperthermia).

Within a period of 20 days from the start of the treatment with the canine diet product in accordance with the present invention, the pathology was considered resolved for 128 of 139 cases, i.e. for about 92% of the subjects.

In particular, in the subjects for whom the enterocolitis pathology is considered resolved, a disappearance of the vomit and an increase of the body weight were in particular found.

### Example 3:

A second group of 48 dogs affected by enteritis were fed for a month with a product for the diet in accordance with the present invention.

The subjects initially had the symptoms of the enteritis pathology (in addition to the abovementioned symptoms normally encountered in the colitis and enterocolitis pathology, the subjects affected by enteritis can have borborygmi, i.e. intestinal noises, anemia, leucopenia, and/or even shock that in the most serious cases can lead to death). In a time period comprised between 1 and 20 days, the symptoms manifested before the start of the treatment disappeared in 39 out of 48 subjects subjected to the diet treatment with the product in accordance with the present invention.

The tests conducted on subjects affected by enteritis have therefore shown that the exclusive administration of the product according to the invention proved resolutive for about 81% of the cases. In particular, in the subjects for whom the resolution of the pathology was verified, there was a significant decrease of the diarrhea with the appearance of solid and compact feces, as well as an increase of appetite and an increase of body weight of the subjects.

The three above-reported examples demonstrate that the exclusive administration of the product for the diet of dogs affected by inflammatory intestinal diseases according to the invention, for a treatment time period of less than a month, more in detail, less than 20 days, was capable of evolving about 90% of the subjects from their initial pathological condition to a state of normal health. Overall, indeed, the pathology was completely overcome by 186 out of 206 subjects subjected to the exclusive diet treatment with the product according to the invention.

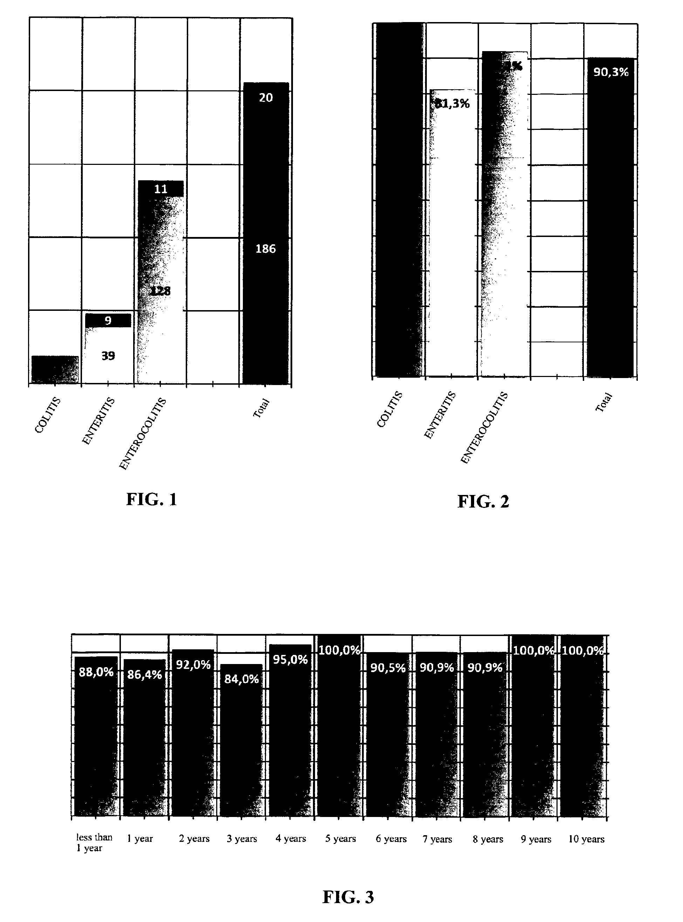

The abovementioned data is summarized in the two enclosed graphs, respectively illustrated in Figure 1 and in Figure 2. More in detail, the graph of Figure 1 reports the number of resolved and unresolved cases (the latter reported in the upper portion with different color of each histogram rectangular) for each of the considered pathologies and for all of the subjects on whom the clinical tests were executed; Figure 2 reports the percentage of cases resolved with only the administration of the canine diet product according to the present invention for the various pathologies considered and for all of the subjects on whom the clinical tests were executed.

In addition, it has been ascertained that the resolution percentage for the various pathologies resulted independent of the race of the dog subjected to the treatment. In addition, the resolution percentages did not seem to be affected by the age of the subjects, as is inferred by the graph reported in Figure 3, in which the resolution percentages are indicated for the subjects as a function of age.

### Example 4:

A further verification was also carried out by substituting, in the diet of several dogs having an inflammatory pathology, after an initial treatment period, the diet product according to the present invention with a modified product, having a formulation entirely similar to that of the product in accordance with the present invention, which however comprised white meat as protein source, in particular chicken meat, rather than, protein hydrolysates.

More in detail, 20 subjects affected by colitis were initially subjected to an exclusive diet treatment with the canine diet product in accordance with the present invention until the disappearance of the symptoms associated with the colitis pathology.

The subjects were then subjected to an exclusive diet treatment with the modified canine diet product as specified above, comprising chicken meat as source of protein rather than protein hydrolysates.

Over 70% of the subjects responded with the recurrence of the colitis symptoms and in particular with the appearance of soft, even liquid feces - which returned to normality with the reintroduction into the diet of the animal diet product according to the present invention, containing protein hydrolysates as source of protein.

The remaining subjects responded in a variable manner, without showing significant recurrence of symptoms, and in particular an evident worsening of the feces consistency such to be considered diarrhea or non-normal feces.

Therefore, it should be deemed that in these final subjects, the acute inflammatory phase - during which the intestine is particular sensitized and hence inclined to develop immune responses that would lead to a recurrence of the inflammation - has already been overcome.

The finding thus conceived therefore attains the pre-established objects.

In the practical achievement thereof, the invention can of course also assume forms and configurations different from that illustrated above, without departing from the present protective scope. In addition, all details can be substituted by technically equivalent elements and the size, shape and materials used can be of any type according to requirements.

## Claims

1. Product for the diet of dogs affected by inflammatory intestinal diseases, which comprises at least one source of dietary fiber and at least one source of proteins, **characterized in that** said at least one dietary fiber source comprises ascophyllum nodosum comprised in said canine diet product in a weight percentage comprised between 15% and 25% and said at least one protein source comprises at least one protein hydrolysate, comprised in said canine diet product in a weight percentage comprised between 18% and 25%.

2. Product for the diet of dogs affected by inflammatory intestinal diseases according to claim 1, **characterized in that** said ascophyllum nodosum is comprised in said canine diet product in a weight percentage equal to about 20%.

3. Product for the diet of dogs affected by inflammatory intestinal diseases according to claim 1, **characterized in that** it has an overall nutritional profile formed by at least proteins, fats, carbohydrates, dietary fiber and ash, in which the proteins are in a weight quantity comprised between 15% and 40%, the fats are in a weight quantity comprised between 5% and 25%, the carbohydrates are in a weight quantity comprised between 18% and 65%, the dietary fiber is in a weight quantity comprised between 15% and 25% and the ash is in a weight quantity less than 10%.

4. Product for the diet of dogs affected by inflammatory intestinal diseases according to claim 3, **characterized in that** at least 80% of said dietary fiber is brought by said ascophyllum nodosum.

5. Product for the diet of dogs affected by inflammatory intestinal diseases according to claim 3 or 4, **characterized in that** at least 5% of said dietary fiber is soluble fiber.

6. Product for the diet of dogs affected by inflammatory intestinal diseases according to claim 1, **characterized in that** said at least one protein hydrolysate comprises at least one fish hydrolysate.

7. Product for the diet of dogs affected by inflammatory intestinal diseases according to any one of the preceding claims, **characterized in that** said at least one protein hydrolysate has a maximum molecular mass of 6,000 Dalton.

8. Product for the diet of dogs affected by inflammatory intestinal diseases according to any one of the preceding claims, **characterized in that** it also comprises at least one prebiotic substance.

9. Product for the diet of dogs affected by inflammatory intestinal diseases according to any one of the preceding claims, **characterized in that** it has a moisture content lower than 15%.

10. Product for the diet of dogs affected by inflammatory intestinal diseases according to claim 9, **characterized in that** it is in the form of croquettes, preferably obtained via extrusion.

## Patentansprüche

1. Produkt für die Ernährung von Hunden mit entzündlichen Darmerkrankungen, das wenigstens eine Ballaststoffquelle und wenigstens eine Proteinquelle umfasst, **dadurch gekennzeichnet, dass** die genannte wenigstens eine Ballaststoffquelle in dem genannten Produkt für die Ernährung von Hunden Ascophyllum nodosum zu einem Gewichtsanteil zwischen 15 % und 25 % enthält und die genannte wenigstens eine Proteinquelle wenigstens ein Proteinhydrolysat umfasst, das in dem genannten Produkt für die Ernährung von Hunden zu einem Gewichtsanteil von 18 % bis 25 % vorhanden ist.

2. Produkt für die Ernährung von Hunden mit entzündlichen Darmerkrankungen nach Anspruch 1, **dadurch gekennzeichnet, dass** das genannte Ascophyllum nodosum in dem genannten Produkt für die Ernährung von Hunden zu einem Gewichtsanteil von ca. 20 % enthalten ist.

3. Produkt für die Ernährung von Hunden mit entzündlichen Darmerkrankungen nach Anspruch 1, **dadurch gekennzeichnet, dass** es insgesamt ein Nährstoffprofil aufweist, das wenigstens aus Proteinen, Fetten, Kohlenhydraten, Ballaststoffen und Asche besteht, bei dem die Proteine einen Gewichtsanteil zwischen 15 % und 40 %, die Fette einen Gewichtsanteil zwischen 5 % und 25 %, die Kohlenhydrate einen Gewichtsanteil zwischen 18 % und 65 %, die Ballaststoffe einen Gewichtsanteil zwischen 15 % und 25 % und die Asche einen Gewichtsanteil von weniger als 10 % ausmachen.

4. Produkt für die Ernährung von Hunden mit entzündlichen Darmerkrankungen nach Anspruch 3, **dadurch gekennzeichnet, dass** mindestens 80 % der genannten Ballaststoffe über das genannte Ascophyllum nodosum zugeführt werden.

5. Produkt für die Ernährung von Hunden mit entzündlichen Darmerkrankungen nach Anspruch 3 oder 4, **dadurch gekennzeichnet, dass** mindestens 5 % der genannten Ballaststoffe aus löslichen Fasern bestehen.

6. Produkt für die Ernährung von Hunden mit entzündlichen Darmerkrankungen nach Anspruch 1, **dadurch gekennzeichnet, dass** das genannte wenigstens eine Proteinhydrolysat wenigstens ein Fischhydrolysat umfasst.

7. Produkt für die Ernährung von Hunden mit entzündlichen Darmerkrankungen nach einem beliebigen der vorangegangenen Ansprüche, **dadurch gekennzeichnet, dass** das genannte wenigstens eine Proteinhydrolysat ein Molekulargewicht von maximal 6.000 Dalton aufweist.

8. Produkt für die Ernährung von Hunden mit entzündlichen Darmerkrankungen nach einem der beliebigen vorangegangenen Ansprüche, **dadurch gekennzeichnet, dass** es mindestens ein Präbiotikum umfasst.

9. Produkt für die Ernährung von Hunden mit entzündlichen Darmerkrankungen nach einem der beliebigen vorangegangenen Ansprüche, **dadurch gekennzeichnet, dass** es einen Feuchtigkeitsgehalt von weniger als 15 % aufweist.

10. Produkt für die Ernährung von Hunden mit entzündlichen Darmerkrankungen nach Anspruch 9, **dadurch gekennzeichnet, dass** es in Form von, vorzugsweise extrudierten, Kroketten vorliegt.

## Revendications

1. Produit pour l'alimentation des chiens atteints de maladies intestinales inflammatoires, lequel comprend au moins une source de fibres alimentaire et au moins une source de protéines, **caractérisé en ce que** ladite au moins source de fibre alimentaire comprend ascophyllum nodosum compris dans ledit produit pour l'alimentation des chiens en pourcentage en poids compris entre 15 % et 25 % et ladite au moins une source de protéines comprend au moins un hydrolysat protéique, compris dans ledit produit pour l'alimentation des chiens en pourcentage en poids compris entre 18 % et 25 %.

2. Produit pour l'alimentation des chiens atteints de maladies intestinales inflammatoires selon la revendication 1, **caractérisé en ce que** ledit ascophyllum nodosum est compris dans ledit produit pour l'alimentation des chiens en pourcentage en poids égal à environ 20 %.

3. Produit pour l'alimentation des chiens atteints de maladies intestinales inflammatoires selon la revendication 1, **caractérisé en ce qu'**il a un profil nutritionnel total constitué d'au moins protéines, graisses, carbohydrates, fibre alimentaire et cendres, dans lequel les protéines sont en quantité en poids comprise entre 15 % et 40 %, les graisses sont en quantité en poids comprise entre 5 % et 25 %, les carbohydrates sont en quantité en poids comprise entre 18 % et 65 %, la fibre alimentaire est en quantité en poids comprise entre 15 % et 25 % et les cendres sont en quantité en poids inférieure à 10 %.

4. Produit pour l'alimentation des chiens atteints de maladies intestinales inflammatoires selon la revendication 3, **caractérisé en ce qu'**au moins 80 % de ladite fibre alimentaire est apportée par ledit ascophyllum nodosum.

5. Produit pour l'alimentation des chiens atteints de maladies intestinales inflammatoires selon la revendication 3 ou 4, **caractérisé en ce qu'**au moins 5 % de ladite fibre alimentaire est fibre soluble.

6. Produit pour l'alimentation des chiens atteints de maladies intestinales inflammatoires selon la revendication 1, **caractérisé en ce qu'**au moins un hydrolysat protéique comprend au moins un hydrolysat de poisson.

7. Produit pour l'alimentation des chiens atteints de maladies intestinales inflammatoires selon une quelconque des revendications précédents, **caractérisé en ce que** ledit au moins un hydrolysat protéique a une masse moléculaire maximale de 6.000 Dalton.

8. Produit pour l'alimentation des chiens atteints de maladies intestinales inflammatoires selon une quelconque des revendications précédentes, **caractérisé en ce qu'**il comprend en outre au moins un prébiotique.

9. Produit pour l'alimentation des chiens atteints de maladies intestinales inflammatoires selon une quelconque des revendications précédentes, **caractérisé en ce qu'**il a une teneur en humidité inférieure à 15 %.

10. Produit pour l'alimentation des chiens atteints de maladies intestinales inflammatoires selon la revendication 9, **caractérisé en ce qu'**il est en forme de croquettes, obtenues, de préférence, par extrusion.
